# FASCICULE DE BREVET EUROPEEN

(11) **EP 0 809 704 B1**
(45) Date de publication et mention de la délivrance du brevet: **20.08.2008**
(21) Numéro de dépôt: 96903077.4
(22) Date de dépôt: 09.02.1996
(51) Int. Cl.: C12N 15/86, C12N 15/34, C12N 15/70, C12N 7/01, C12N 1/21, A61K 35/76

(54) **PROCEDE DE PREPARATION DE GENOME D'ADENOVIRUS RECOMBINANTS**
VERFAHREN ZUR HERSTELLUNG REKOMBINANTER ADENOVIRUS-GENOME
METHOD FOR PREPARING A RECOMBINANT ADENOVIRUS GENOME

(30) Priorité: 13.02.1995 FR 9501632
(43) Date de publication de la demande: 03.12.1997
(73) Titulaire: CENTELION, 94400 Vitry Sur Seine (FR)
(72) Inventeur: CROUZET, Joel, 92330 Sceaux (FR); NAUDIN, Laurent, 45370 Clery-Saint-André (FR); ORSINI, Cécile, 75012 Paris (FR); VIGNE, Emmanuelle, 94200 Ivry-sur-Seine (FR); YEH, Patrice, 75005 Paris (FR)
(74) Mandataire: Bouvet, Philippe
(86) Numéro de dépôt international: PCT/FR1996/000215
(87) Numéro de publication internationale: WO 1996/025506

(56) Documents cités:
- WO-A-95/00655
- WO-A-95/02697
- WO-A-95/03400
- WO-A-96/13596
- WO-A-96/13597
- WO-A-96/17070
- GENE, vol. 50, no. 1-3, 1986, ELSEVIER SCIENCE PUBLISHERS,B.V.,AMSTERDAM,NL;, pages 161-171, XP002004335 G. GHOSH-CHOUDHURY ET AL.: "Human adenovirus cloning vectors based on bacterial plasmids" cité dans la demande
- HUMAN GENE THERAPY, vol. 4, 1993, MARY ANN LIEBERT, INC. PUBLISHERS, NEW YORK, US, pages 461-476, XP002004336 D.P. RICH ET AL.: "Development and analysis of recombinant adenoviruses for gene therapy of cystic fibrosis" cité dans la demande
- PROC. NATL.ACAD SCI., vol. 91, no. 13, 13 Septembre 1994, NATL. ACAD SCI.,WASHINGTON,DC,US;, pages 8802-8806, XP002004337 A.J. BETT ET AL.: "An efficient and flexible system for construction of adenovirus vectors with insertions or deletions in early regions 1 and 3"
- EMBO J., vol. 3, no. 12, 1984, OXFORD UNIVERSITY PRESS,GB;, pages 2917-2922, XP002004338 F.L. GRAHAM : "covalently closed circles of human Adenovirus DNA are infectious" cité dans la demande
- NUCLEIC ACIDS RESEARCH, vol. 17, no. 8, IRL PRESS LIMITED,OXFORD,ENGLAND, pages 3037-3048, XP002004339 G. KETNER ET AL.: "Complementation of adenovirus E4 mutants by transient expression of E4 cDNA and deletion plasmids"
- PROC. NATL.ACAD SCI., vol. 81, Octobre 1984, NATL. ACAD SCI.,WASHINGTON,DC,US;, pages 6290-6294, XP002004340 V. NATARAJAN ET AL.: "Proximal and distal domains that control in vitro transcription of the adenoviral IVa2 gene"

## Description

La présente invention concerne un nouveau procédé de préparation d'adénovirus recombinants et l'utilisation de ces adénovirus en thérapie génique. Elle concerne également des plasmides procaryotes adaptés à la préparation de ces adénovirus.

La thérapie génique consiste à corriger une déficience ou une anomalie (mutation, expression aberrante, etc.) par introduction d'une information génétique dans la cellule ou l'organe affecté. Cette information génétique peut être introduite soit in vitro dans une cellule extraite de l'organe, la cellule modifiée étant alors réintroduite dans l'organisme. soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines population. cellulaires. En particulier, les rétrovirus (RSV, HMS, MMS, etc.), le virus HSV, les virus adéno-associés, et les adénovirus.

Parmi ces virus, les adénovirus présentent certaines propriétés intéressantes pour une utilisation en thérapie génique. Notamment, ils ont un spectre d'hôte assez large, sont capables d'infecter des cellules quiescentes, ne s'intègrent pas au génome de la cellule infectée, et n'ont pas été associés à ce jour à des pathologies importantes chez l'homme. Les adénovirus ont ainsi été utilisés pour transférer des gènes d'intérêt dans le muscle (Ragot et al., Nature 361 (1993) 647), le foie (Jaffe et al., Nature genetics 1 (1992) 372), le système nerveux (Akli et al., Nature genetics 3 (1993) 224), etc.

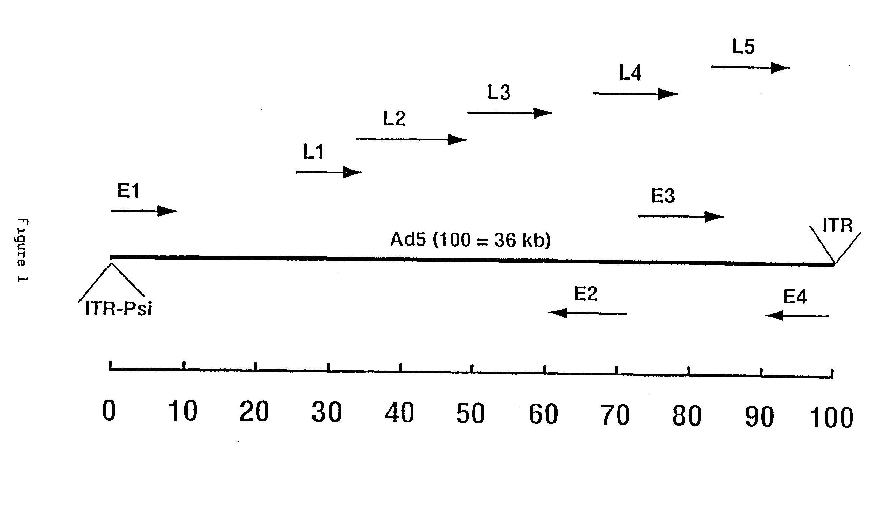

Les adénovirus sont des virus à ADN double brin linéaire d'une taille de 36 kb environ. Leur génome comprend notamment une séquence inversée répétée (ITR) à chaque extrémité, une séquence d'encapsidation (Psi), des gènes précoces et des gènes tardifs (Cf figure 1). Les principaux gènes précoces sont contenus dans les régions E1, E2, E3 et E4. Parmi ceux-ci, les gènes contenus dans la région E1 sont nécessaires à la propagation virale. Les principaux gènes tardifs sont contenus dans les régions L1 à L5. Le génome de l'adénovirus Ad5 a été entièrement séquencé et est accessible sur base de données (voir notamment Genebank M73260). De même des parties, voire la totalité d'autres génomes d'adénoviraux (Ad2, Ad7, Ad12, etc.) ont également été séquencées.

Compte tenu des propriétés des adénovirus mentionnées ci-dessus, et compte tenu du fait qu'il est possible d'obtenir des titres viraux élevés, ceux-ci ont déjà été utilisés pour le transfert de gènes in vivo. A cet effet, différents vecteurs dérivés des adénovirus ont été préparés, incorporant différents gènes (β-gal, OTC, a-1AT, cytokines, etc.). Dans chacune de ces constructions, l'adénovirus a été modifié de manière à le rendre incapable de réplication après transfert génique. Ainsi, les constructions décrites dans l'art antérieur sont des adénovirus délétés des régions E1 et éventuellement E3 au niveau desquelles sont insérées les séquences d'ADN hétérologue (Levrero et al., Gene 101 (1991) 195; Gosh-Choudhury et al., Gene 50 (1986) 161). D'autres constructions comportent une délétion au niveau de la région E1 et d'une partie non essentielle de la région E4 (WO94/12649), ou une organisation génomique modifiée (FR 94 13355).

Cependant, l'exploitation industrielle et thérapeutique des adénovirus est encore limitée par les méthodes actuelles de préparation de ces virus recombinants.

Les adénovirus sont en effet produits par transfection de l'ADN du virus recombinant dans une lignée cellulaire d'encapsidation compétente. Il peut s'agir d'une transfection simple, lorsque l'on peut disposer d'une construction portant l'ensemble du génome du virus recombinant, ou, comme c'est le cas le plus souvent, d'une co-transfection de plusieurs fragments d'ADN apportant les différentes parties du génome viral recombinant. Dans ce cas, le procédé implique une ou plusieurs étapes de recombinaison homologue entre les différentes constructions dans la lignée cellulaire d'encapsidation, pour générer l'ADN du virus recombinant. Pour la mise en oeuvre de l'une ou l'autre de ces méthodes, il est donc nécessaire de disposer des constructions appropriées, portant l'ensemble ou des parties du génome de l'adénovirus recombinant que l'on souhaite produire.

Il existe différentes méthodes décrites dans l'art antérieur pour la préparation de ces constructions in vitro. La technique la plus généralement utilisée consiste à isoler l'ADN viral puis à le modifier in vitro par les méthodes classiques de biologie moléculaire (digestion, ligature, etc.). Les constructions obtenues sont ensuite purifiées et utilisées pour transfecter les lignées d'encapsidation. Toutefois, cette technique implique la production de stocks de virus et la purification d'ADN viral pour chaque construction ou pour toute manipulation de l'ADN du virus recombinant. Une autre technique repose sur l'utilisation d'un plasmide portant une partie du gènome du virus recombinant, qui est co-transfecté avec un virus apportant la partie manquante du gènome. Néanmoins, comme indiqué ci-avant, cette méthode implique une recombinaison dans la lignée d'encapsidation et la disposition d'un virus supplémentaire approprié. Pour remédier à ces inconvénients, il a été proposé d'utiliser des plasmides procaryotes pour préparer les ADN viraux utilisables pour la transfection. En particulier, Bett et al. (PNAS 91 (1994) 8802) décrit la construction d'un plasmide réplicatif chez E.coli comportant un génome adénoviral modifié (plasmide pBHG10). Plus précisément, ce plasmide porte un génome adénoviral délété des régions E1, E3 et Psi, circularisé par jonction des séquences TTR, et qui comprend, insérée au niveau de la région 188-1339 du génome de l'adénovirus, une partie du plasmide pBR322. Ce plasmide peut être répliqué chez E. Coli, manipulé pour l'insertion de gènes d'intérêt, mais il présente toujours des inconvénients. Son utilisation pour la production de virus implique notamment l'emploi d'un second plasmide au moins apportant la région gauche du génome viral. D'autres plasmides de ce type et présentant le même genre d'inconvénients ont été décrits par exemple par Graham (EMBOJ. 3(12) (1984) 2917). Ces plasmides nécessitent aussi de procéder à une étape de recombinaison dans les cellules d'encapsidation. D'autres plasmides ont été décrits dans Rich et al. (Human Gene Ther. 4 (1993) 461), WO 96/17070 et WO 96/13597.

En particulier ces technologies nécessitent l'utilisation de différents plasmides pour permettre la manipulation dans différentes régions du génome adénoviral. De plus l'obtention du virus recombinant n'a lieu qu'après recombinaison homologue des fragments génomiques co-transfectés dans les cellules d'encapsidation. Ceci limite d'autant la fréquence d'obtention des virus recombinants et le processus global est lent.

Il existe donc dans l'art antérieur un besoin clair de pouvoir disposer de plasmides appropriés, facilement manipulables et amplifiables in vitro, pour la préparation de génomes adénoviraux recombinants. Il est également important que les génomes ainsi produits soient pratiquement dépourvus de régions provenant du plasmide, qui sont susceptibles (i) d'induire une réponse immunitaire (ii) de coder pour des protéines de résistance et (iii) de réduire la capacité du virus en tant que vecteur.

La présente invention permet de remédier à ces inconvénients. La présente invention décrit en effet des plasmides répondant à toutes ces exigences et permettant ainsi une production clonale rapide et efficace d'adénovirus recombinants utilisables thérapeutiquement.

Plus particulièrement, un premier objet de l'invention réside dans un plasmide procaryote comprenant un génome d'adénovirus recombinant bordé par un ou plusieurs sites de restriction non présent dans ledit génome.

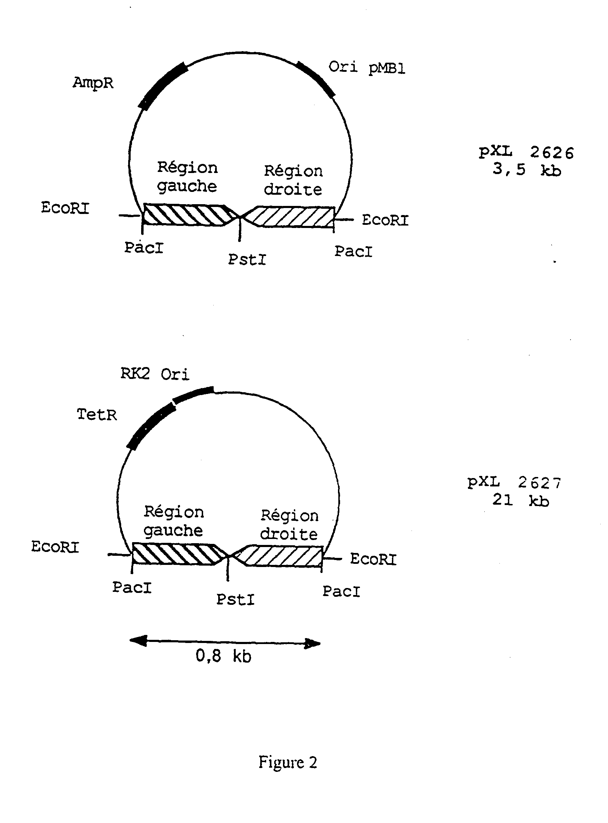

Un tel plasmide est représenté par exemple sur la figure 2.

Le génome d'adénovirus recombinant présent dans les plasmides selon l'invention est avantageusement un génome complet. Ceci est particulièrement intéressant puisque cela permet de s'affranchir de l'emploi d'une deuxième construction apportant une autre partie du génome viral, et de l'étape de recombinaison dans la lignée d'encapsidation. Un autre avantage des plasmides selon l'invention provient du fait que le génome adénoviral n'est pas interrompu par des régions du plasmide procaryote. De ce fait, les génomes produits ne contiennent pas de régions du plasmide dont les inconvénients ont été mentionnés ci-avant. Par ailleurs, dans les plasmides selon l'invention, les ITR du génome adénoviral ne sont pas jointes, ce qui permet d'obtenir des ADN viraux linéaires, directement utilisables pour produire les virus recombinants.

De manière plus préférentielle, les plasmides selon l'invention comprennent donc une première région permettant la réplication dans les cellules procaryotes et une deuxième région comportant le génome adénoviral bordé d'un ou plusieurs sites de restriction non présent dans ledit génome.

Plus préférentiellement, les plasmides selon l'invention comprennent également une région permettant la sélection des cellules procaryotes contenant ledit plasmide. Cette région peut être constituée notamment par tout gène conférant la résistance à un produit, et notamment à un antibiotique. Ainsi, on peut citer les gènes conférant une résistance à la kanamycine (Kan^{r}), à l'ampicilline (Amp,^{r}), à la tétracycline (tet^{r} ou à la spectinomycine, par exemple, qui sont couramment utilisés en biologie moléculaire (Maniatis et al., 1989). La sélection de plasmides peut se faire par d'autres gènes que des gènes codant pour des marqueurs de résistance à un antibiotique. D'une manière générale, il s'agit d'un gène qui donne à la bactérie une fonction qu'elle ne possède plus (cela peut correspondre à un gène qui a été délété sur le chromosome ou rendu inactif), le gène sur le plasmide rétablissant cette fonction. A titre d'exemple il peut s'agir d'un gène d'un ARN de transfert qui rétablit une fonction chromosomique déficiente (Somoes et al., 1991).

La région permettant la réplication dans les cellules procaryotes utilisée dans les plasmides de l'invention peut être toute origine de réplication fonctionnelle dans les cellules choisies. Il peut s'agir d'une origine de réplication issue d'un plasmide du groupe d'incompatibilité P (exemple = pRK290) qui permet la réplication dans les souches d'E. Coli pol A. Plus généralement, il peut s'agir de toute origine de réplication issue d'un plasmide se répliquant dans les cellules procaryotes. Ce plasmide peut être un dérivé de pBR322 (Bolivar et al., 1977), un dérivé de pUC (Viera et Messing, 1982), ou d'autres plasmides dérivant du même groupe d'incompatibilité, c'est à dire de ColE1 ou de pMB1 par exemple. Ces plasmides peuvent être choisis par ailleurs dans d'autres groupes d'incompatibilité se répliquant chez Escherichia coli. Il peut s'agir de plasmides dérivés de plasmides appartenant aux groupes d'incompatibilité A, B, FI, FII, FIII, FIV, H1, H11, I1, I2, J, K, L, N, OF, P, Q, T, U, W, X, Y, Z ou 9 par exemple. D'autres plasmides peuvent encore être utilisés, parmi lesquels des plasmides ne se répliquant pas chez E. coli mais chez d'autres hôtes tels que B. subtilis, Streptomyces, P. putida, P. aeruginosa, Rhizobium meliloti, Agrobacterium tumefaciens, Staphylococcus aureus, Streptomyces pristinaespiralis, Enterococcus faecium ou Clostridium. A titre préférentiel, on utilise les origines de réplication issues de plasmides se répliquant chez E. coli.

Comme indiqué précédemment, le génome adénoviral présent dans les plasmides de l'invention est avantageusement un génome complet ou fonctionnel, c'est-à-dire ne nécessitant pas l'apport d'autres régions par recombinaison ou ligature pour la production des stocks viraux dans les lignées d'encapsidation choisies.

Préférentiellement, le génome adénoviral recombinant comprend au moins des séquences ITR et une séquence permettant l'encapsidation.

Les séquences inversées répétées (ITR) constituent l'origine de réplication des adénovicus. Elles sont localisées aux extrémités du génome viral (Cf figure 1), d'où elles peuvent être isolées aisément selon les techniques classiques de biologie moléculaire connues de l'homme du métier. La séquence nucléotidique des séquences ITR des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence ITR gauche correspond à la région comprenant les nucléotides 1 à 103 du génome.

La séquence d'encapsidation (également désignée séquence Psi) est nécessaire à l'encapsidation du génome viral. Elle est localisée dans le génome des adénovirus sauvages, entre l'ITR gauche et la région E1 (Cf figure 1). Elle peut être isolée ou synthétisée artificiellement par les techniques classiques de biologie moléculaire. La séquence nucléotidiques de la séquence d'encapsidation des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, une séquence d'encapsidation fonctionnelle est comprise entre les nucléotides 194 et 358 du génome.

Dans un mode de réalisation préféré de l'invention, le génome de l'adénovirus utilisé est dépourvu de tout ou partie de la région E1. La région E1 est en effet essentielle à la réplication virale et son inactivation conduit à la formation de virus défectifs pour la réplication, c'est-à-dire incapables de se répliquer de façon autonome après transfert génique in vivo. La région E1, ou tout autre région virale considérée, peut être rendue non fonctionnelle par toute technique connue de l'homme du métier, et notamment par suppression totale, substitution, délétion partielle, ou addition d'une ou plusieurs bases dans le ou les gènes considérés. De telles modifications peuvent être facilement réalisées directement sur les plasmides de l'invention, par exemple, au moyens des techniques du génie génétique. Avantageusement, le génome de l'adénovirus utilisé est dépourvu d'une partie de la région E1 comprise entre les nucléotides 454 à 3328 (fragment PvuII-BgIII) ou 382 à 3446 (fragment HinfII-Sau3A).

Selon un mode de réalisation particulièrement avantageux, le génome de l'adénovirus utilisé est également dépourvu de tout ou partie de la région E3 et/ou E4 et/ou IVA2. La demanderesse a maintenant montré qu'il est possible de construire des virus portant ces différents types de délétions. Ces délétions supplémentaires permettent d'accroître la sécurité du vecteur et d'augmenter sa capacité.

Préférentiellement, le génome adénoviral est dépourvu d'une partie de la région E4 comprenant au moins les phases ORF3 et ORF6. Le génome adénoviral peut également être modifié comme décrit dans la demande FR9413355 , de manière à éviter les risques de contamination par des particules de réplication.

Préférentiellement, le génome adénoviral recombinant contient en outre une acide nucléique d'intérêt. L'acide nucléique d'intérêt peut être inséré en différents sites du génome de l'adénovirus. Avantageusement, il est inséré au niveau de la région E1, E3 ou E4. Cependant, il est clair que d'autres sites peuvent être utilisés. En particulier, l'accès à la séquence nucléotidique du génome permet à l'homme du métier d'identifier des régions permettant d'insérer l'acide nucléique d'intérêt.

L'acide nucléique d'intérêt peut être toute séquence d'ADN introduite dont le transfert et/ou l'expression dans la cellule cible est recherchée.

En particulier, il peut comporter un ou plusieurs gènes thérapeutiques et/ou un ou plusieurs gènes codant pour des peptides antigéniques.

Les gènes thérapeutiques qui peuvent ainsi être transférés sont tout gène dont la transcription et éventuellement la traduction dans la cellules cible génèrent des produits ayant un effet thérapeutique.

Il peut s'agir d'un produit homologue vis-à-vis de la cellule cible (c'est-à-dire un produit qui est normalement exprimé dans la cellule cible lorsque celle-ci ne présente aucune pathologie). Dans ce cas, l'expression permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit peut également être hétérologue vis-à-vis de la cellule cible. Dans ce cas, une protéine exprimée peut par exemple compléter ou apporter une activité déficiente dans la cellule lui permettant de lutter contre une pathologie.

Parmi les produits thérapeutiques, on peut citer plus particulièrement les enzymes, les dérivés sanguins, les hormones, les lymphokines : interleukines, interférons, TNF, etc (WO93/19191), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc: les apolipoprotéines : ApoAI, ApoAIV, ApoE, etc (WO94/25073), la dystrophine ou une minidystrophine (FR 9111947), les gènes suppresseurs de tumeurs : p53, Rb, Rap1A, DCC, k-rev, etc (WO9424297), les gènes codant pour des facteurs impliqués dans la coagulation : Facteurs VII, VIII, IX, les gènes suicide (TK, etc), etc.

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Comme indiqué plus haut, l'acide nucléique d'intérêt peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, l'acide nucléique d'intérêt comprend également des séquences permettant l'expression du gène thérapeutique et/ou du gène codant pour le peptide antigénique dans la cellule infectée. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence. Enfin, l'acide nucléique d'intérêt peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Les plasmides selon l'invention peuvent être construits en utilisant des adénovirus d'origine diverse. Différents sérotypes d'adénovirus, dont la structure et les propriétés varient quelque peu, ont en effet été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO 94/26914). Parmi les adénovirus d'origine animale utilisables dans le cadre de la présente invention on peut citer les adénovirus d'origine canine, bovine, murine, (exemple : Mavl, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple].

Selon un mode particulier de réalisation de l'invention, l'adénovirus utilisé est un adénovirus d'origine humaine. Selon un autre mode avantageux, l'adénovirus est un adénovirus d'origine animale.

Comme indiqué ci-avant, le génome d'adénovirus recombinant est avantageusement bordé d'un ou plusieurs sites de restriction absents dudit génome. Ce ou ces sites permettent d'exciser de manière simple et efficace le génome adénoviral recombinant du plasmide. La séquence génomique de l'adénovirus étant connue et accessible, l'homme du métier peut sélectionner par des expériences de routine des sites de restriction absent de ce génome. A titre d'exemple, on peut citer les sites PacI. NspV et SwaI (pour Ad5) ou SnabI (dans Ad2). Il est également possible de rendre certains sites uniques en modifiant la séquence du génome adénoviral. Ainsi, d'autres enzymes peuvent être utilisées si les sites de restriction correspondants sont supprimés modifiés ou délétés dans la séquence adénovirale construite chez E. coli. Les sites peuvent être positionnés directement à coté des extrémités du génome adénoviral, ou espacées de quelques paires de bases.

Un plasmide particulièrement préféré au sens de l'invention est le plasmide pXL2638 comprenant l'origine de réplication du plasmide RK2, le gène de résistance à la tétracycline et un génome d'adénovirus recombinant possédant des régions E1 et E3 non fonctionnelles et bordé de 2 sites Pac I. Un acide nucléique d'intérêt peut être inséré en différents sites du génome adénoviral, et notamment au niveau des régions E1, E3 et E4. Différents sites de restriction peuvent être utilisés à cet effet, tel que notamment le site XbaI.

Les plasmides selon la présente invention peuvent être construits de différentes manières. Selon une méthode préférée, on construit dans un premier temps des fragments portant les ITR du génome adénoviral, bordés du ou des sites de restriction appropriés. Ces ITR sont ensuite introduites dans un plasmide procaryote, puis, dans une troisième étape, le génome adénoviral recombinant est reconstruit entre les ITR, soit par ligature, soit par recombinaison. De manière préférée, le génome est reconstruit par recombiaison entre le génome adénoviral recombinant désiré et les régions homologues (comprenant les ITR et les régions flanquantes) du plasmide.

Plus particulièrement, la reconstruction du génome peut être réalisée chez E. coli en utilisant une souche polA afin de sélectionner les événements de recombinaison homologue. Il est évident que ces constructions peuvent aussi être réalisées en l'absence de systèmes permettant de sélectionner des événements de recombinaison. En effet de tels événements de recombinaison peuvent être criblés par minipréparation, perte ou acquisition d'un marqueur, ou bien criblage à l'aide de sondes radioactives spécifiques pour les jonctions obtenues ou perdues. De plus, il existe d'autres techniques permettant de sélectionner des événements de recombinaison homologue chez E. coli. Parmi celles -ci, citons l'utilisation de plasmides thermosensibles pour leur réplication (Hamilton et al., 1989), l'utilisation de molécules circulaires non réplicatives (décrite par exemple par Slater et Maurer, 1993), l'utilisation de souches dans lesquelles le vecteur utilisé ne se réplique pas (Miller et Mekalanos. 1988. Zeef et al., 1994), etc. Tous ces systèmes peuvent être utilisés à la place de souches polA et d'une transformation avec un plasmide dérivé de pBR322, ou ses nombreux dérivés, ou d'autres plasmides à réplication PoIA-dépendante ou bien des plasmides ne se réplicant pas chez Escherichia coli.

Un autre objet de la présente demande concerne toute cellule procaryote contenant un plasmide tel que défini ci-avant. Il peut s'agir en particulier de toute bactérie pour laquelle il existe un système de vecteur où l'ADN recombinant peut être introduit. Citons par exemple Escherichia coli, Salmonella typhimurium, Bacillus subtilis, Pseudomonas putida, Pseudomonas aeruginosa, Agrobacterium tumefaciens. Rhizobium meliloti ou les bactéries du genre Streptomyces. Ces cellules sont obtenues avantageusement par transformation selon les techniques connues de l'homme du métier. La transformation peut notamment être effectuée par la technique de transformation au CaCl₂ (Dagert et Ehrlich, 1979), ou celle mise au point par Hanahan et al. (1983) ou toute technique dérivée de celle-ci (Maniatis et al., 1989), ainsi que par électrotransformation (Wirth et al., 1989). Voir également les techniques générales de Biologie Moléculaire ci-après.

Un autre objet de la présente invention réside dans un procédé de production de génomes d'adénovirus recombinants. Selon ce procédé, des cellules procaryotes telles que décrites ci-dessus sont cultivées puis, dans une deuxième étape, les plasmides sont récupérés. Avantageusement, la culture est réalisée pendant un temps suffisamment long pour produire des quantités appropriées de plasmide. Le plasmide peut être récupéré par toute technique connue de l'homme du métier pour la préparation d'ADN plasmidique. Ainsi, il peut être récupéré par préparation d'un lysat clair suivi d'une centrifugation dans un gradient en chlorure de césium (Maniatis et al., 1989). D'autres techniques peuvent être utilisées, faisant appel à d'autres méthodes de lyse utilisant le triton X-100, par exemple (Ausubel et al., 1987), ou bien une colonne d'échange d'anions après l'étape de lyse et de séparation de l'ADN plasmidique vis à vis de la majorité de l'ADN chromosomique et des protéines. Les plasmides ainsi récupérés peuvent ensuite être purifiés et traités en présence de l'enzyme de restriction correspondant aux sites bordant le génome viral. Ceci permet en une seule étape de générer un génome d'adénovirus recombinant linéaire, directement utilisable pour la production clonale de virus recombinants.

A cet égard, une première méthode pour préparer les virus recombinants consiste à transfecter le génome viral produit à partir des plasmides de l'invention dans une lignée cellulaire d'encapsidation compétente, c'est-à-dire portant en trans toutes les fonctions nécessaires à la complémentation du virus défectif. Ces fonctions sont préférentiellement intégrées dans le génome de la cellule, ce qui réduit les risques de recombinaison, et confère une stabilité accrue à la lignée cellulaire.

Une seconde approche consiste à co-transfecter dans une lignée cellulaire apppropriée le génome recombinant préparé et l'ADN d'un ou de plusieurs virus ou plasmide helper. Selon cette méthode, il n'est pas nécessaire de disposer d'une lignée cellulaire compétente capable de complémenter toutes les fonctions défectives de l'adénovirus recombinant. Une partie de ces fonctions est en effet complémentée par le

ou les virus helper. Ce ou ces virus helper sont eux-mêmes défectifs.

Parmi les lignées cellulaires utilisables, on peut citer notamment la lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %). La transfection peut être réalisée avantageusement directement avec le produit de digestion du plasmide obtenu selon le procédé décrit ci-avant, sans étape de purification du génome adénoviral.

La présente invention concerne également toute composition pharmaceutique comprenant un ou plusieurs adénovirus recombinants préparé selon ce procédé. Les compositions pharmaceutiques de l'invention peuvent être formulées en vue d'une administration par voie topique, orale, parentérale, intranasale, intraveineuse, intramusculaire, sous-cutanée, intraoculaire, transdermique, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution de virus, et est déterminé par infection d'une culture cellulaire appropriée, et mesure, généralement après 15 jours, du nombre de plages de cellules infectées. Les techniques de détermination du titre pfu d'une solution virale sont bien documentées dans la littérature.

Selon la séquence d'ADN hétérologue insérée, les adénovirus de l'invention peuvent être utilisés pour le traitement ou la prévention de nombreuses pathologies, incluant les maladies génétiques (dystrophie, fibrose cystique, etc), les maladies neurodégénératives (alzheimer, parkinson, ALS, etc), les cancers, les pathologies liées aux désordres de la coagulation ou aux dyslipoprotéinémies, les pathologies liées aux infections virales (hépatites, SIDA. etc), etc.

La présente invention sera plus complètement décrite à l'aide des exemples qui suivent, qui doivent être considérés comme illustratifs et non limitatifs.

### LEGENDE DES FIGURES

**Figure 1** : Organisation génétique de l'adénovirus Ad5
**Figure 2** : Cartes de restriction de pXL2626 et pXL2627.
   Amp^{r} : gène de résistance à l'ampicilline; pMB1 Ori, origine de réplication de pMB1;
   Tet^{r}, gène de résistance à la tétracycline; RK2 Ori, origine de réplication du plasmide RK2. Les parties circulaires des cartes des plasmides ne sont pas à la même échelle que celle de 0.8 kb indiquée pour les 2 ITR.

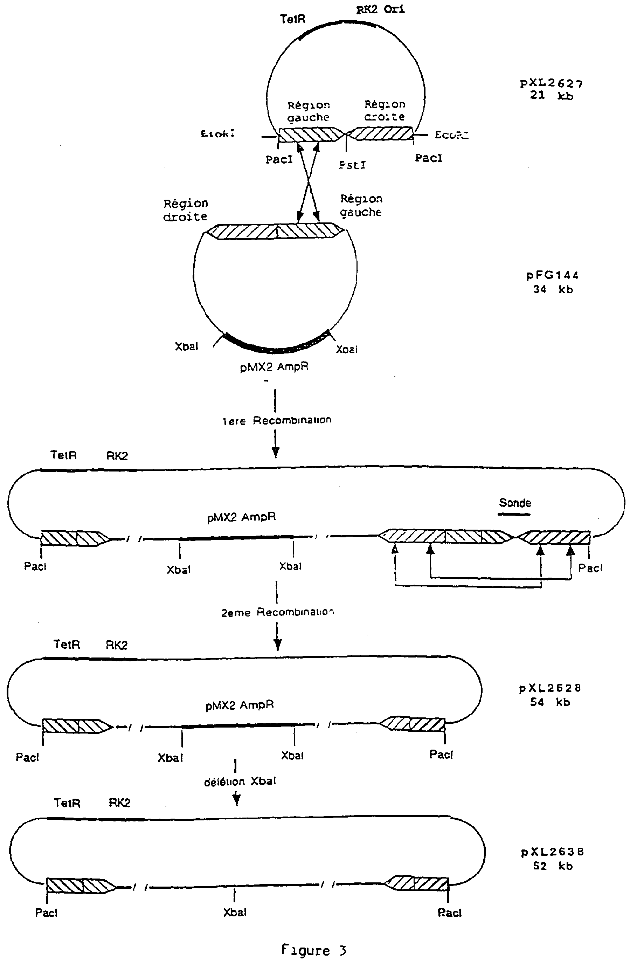

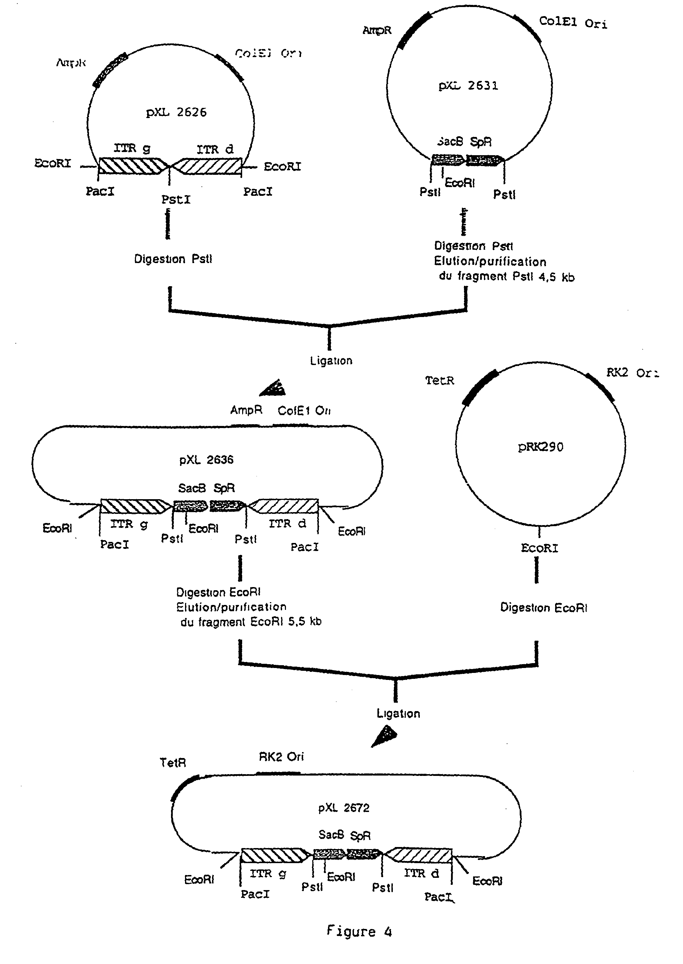

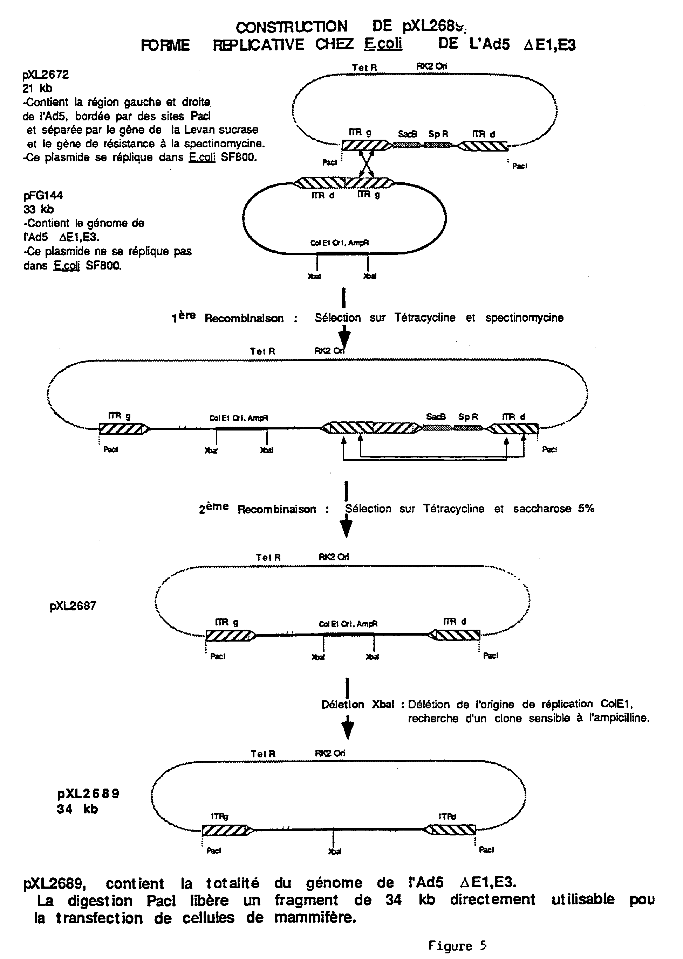

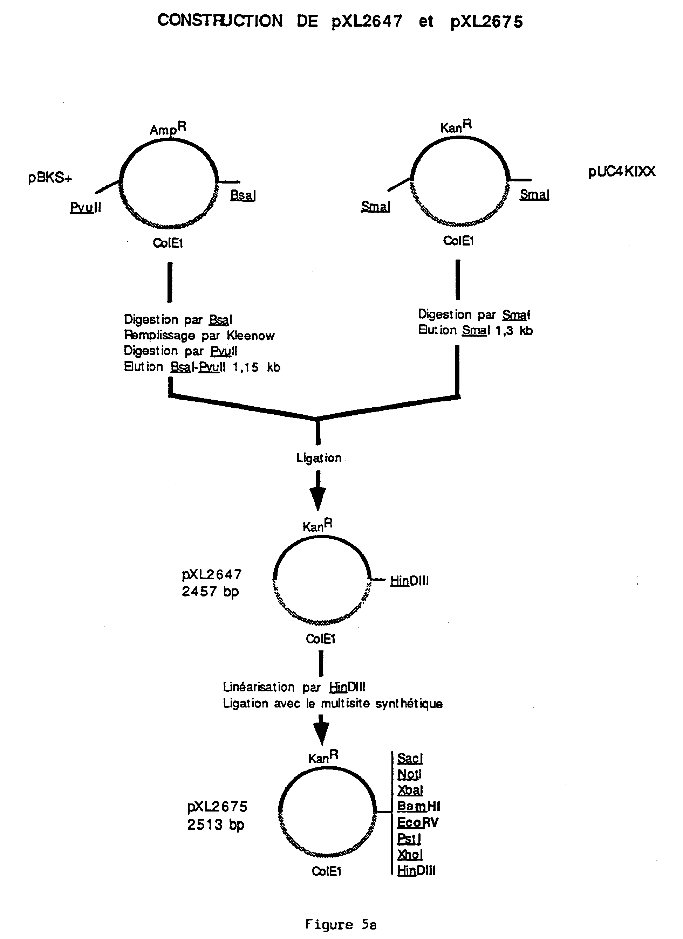

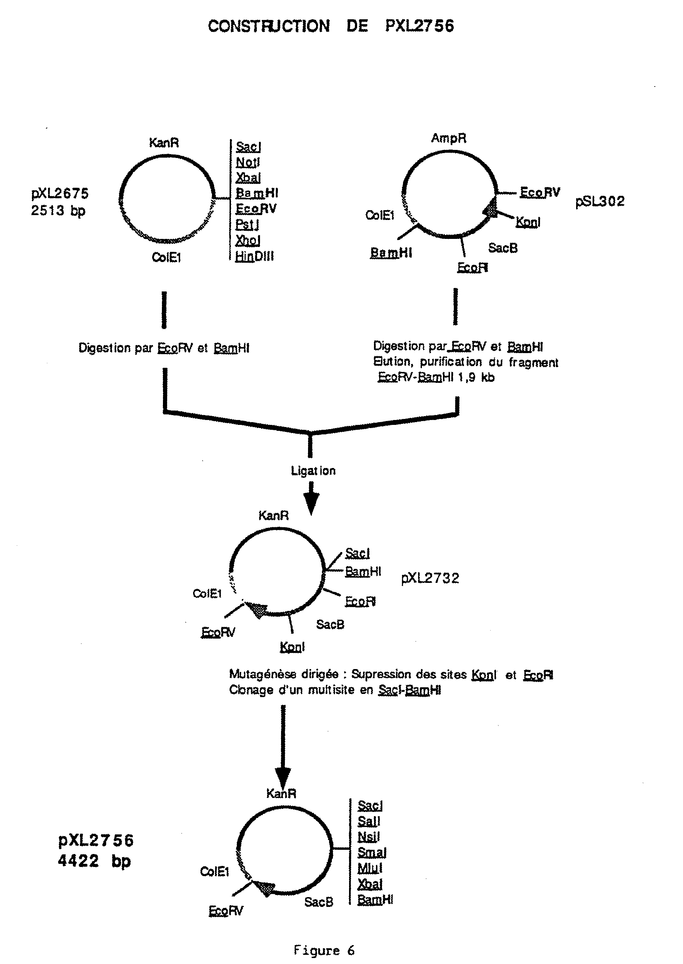

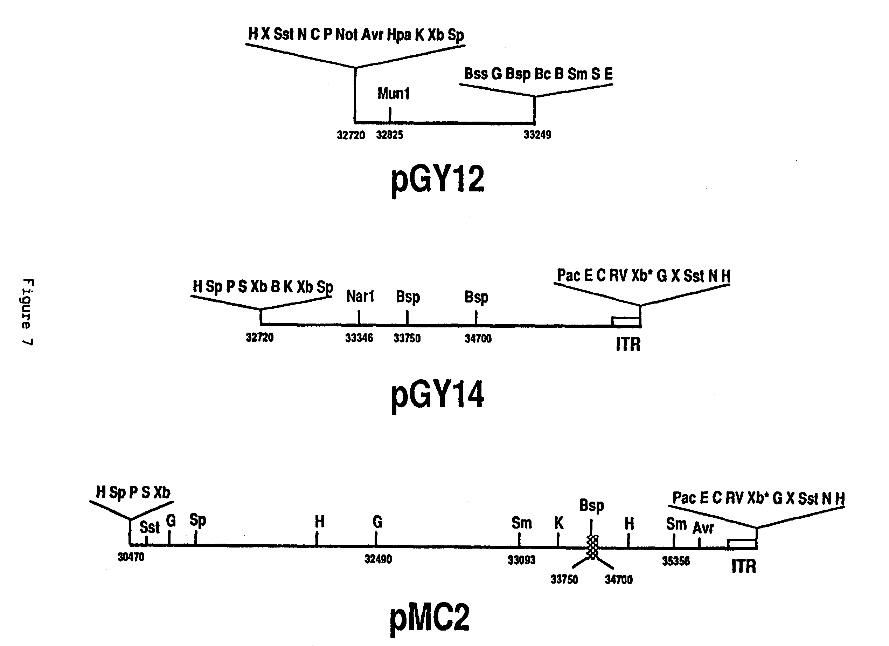

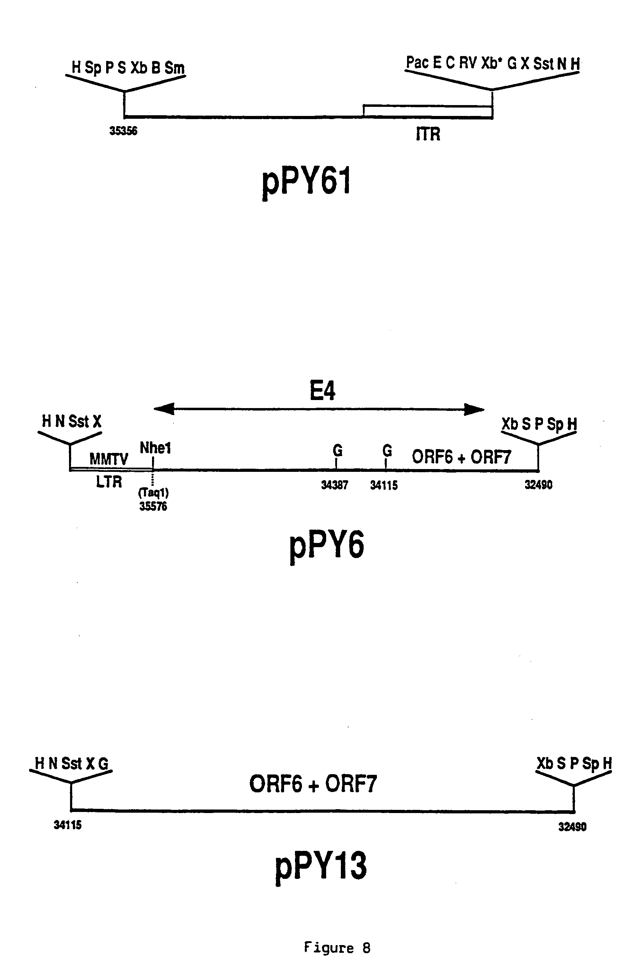

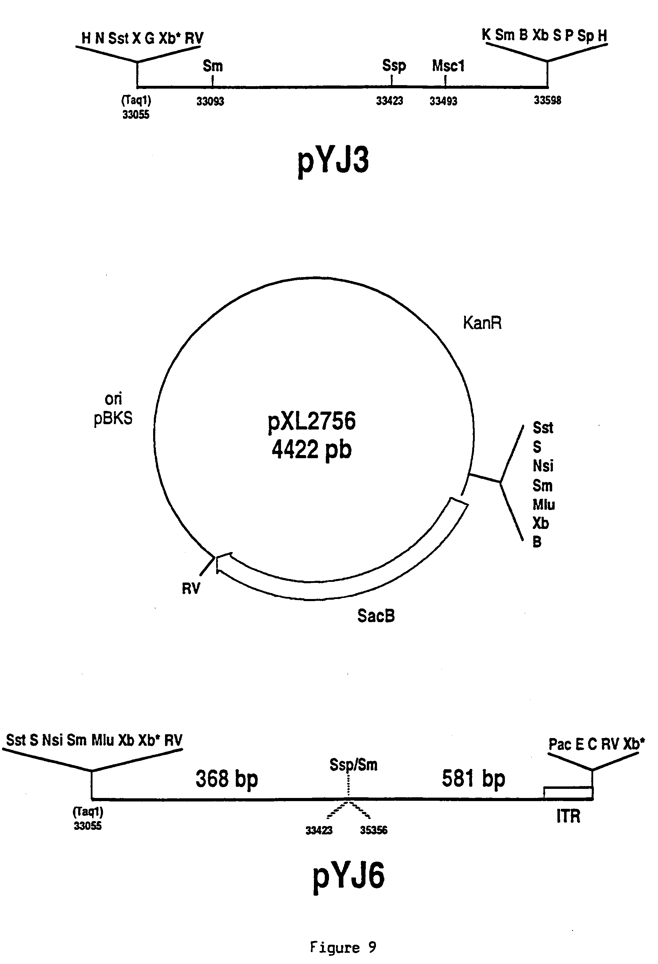

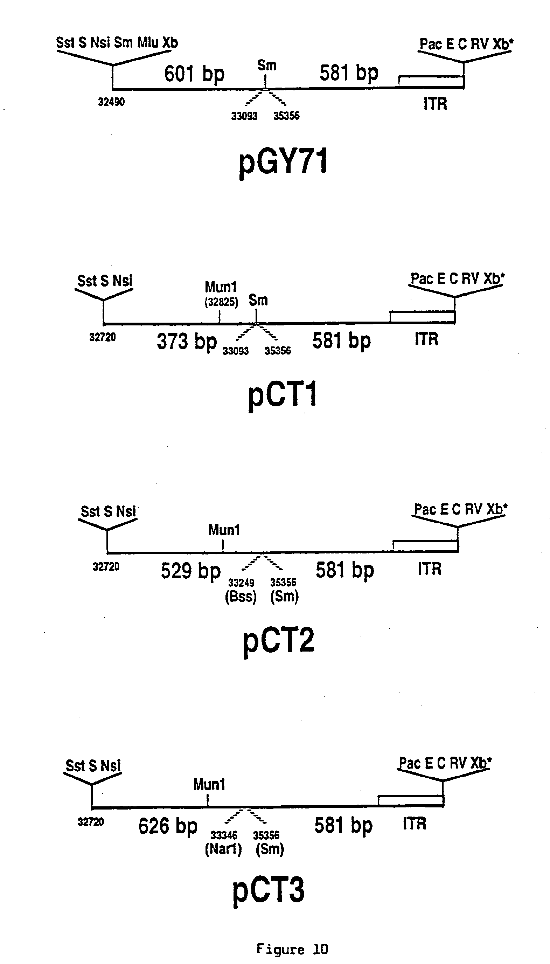

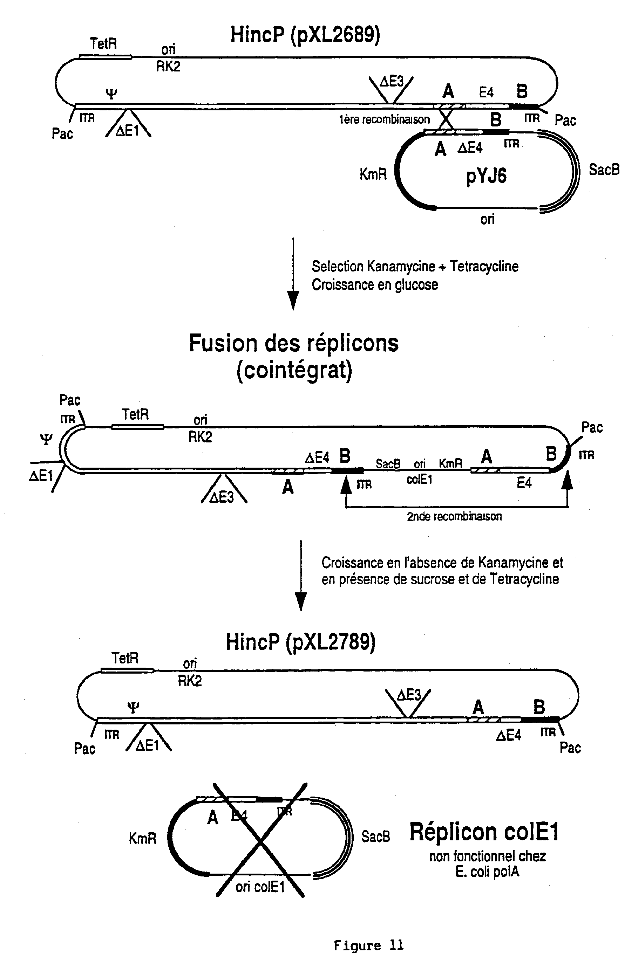

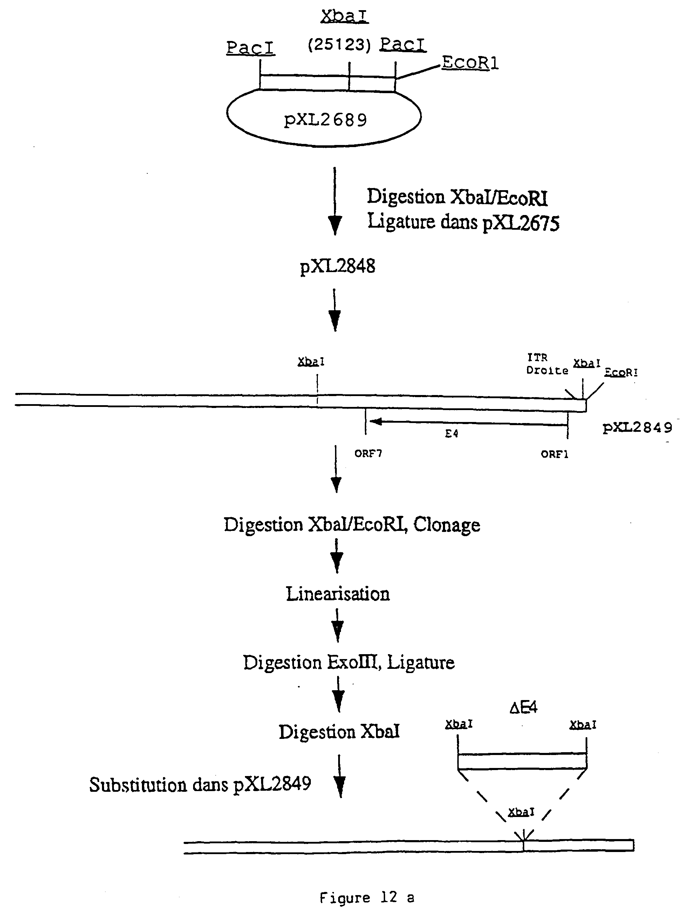

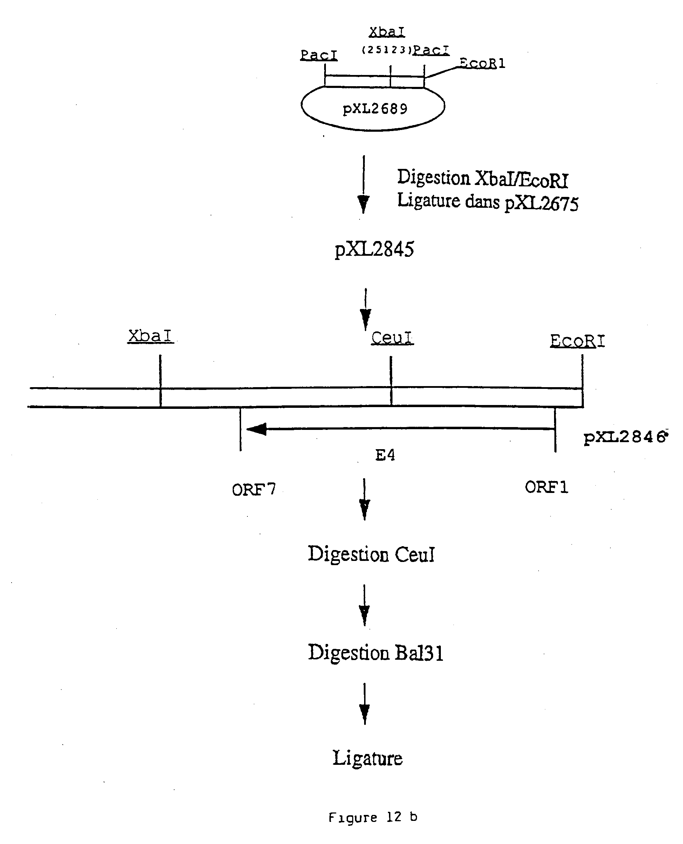

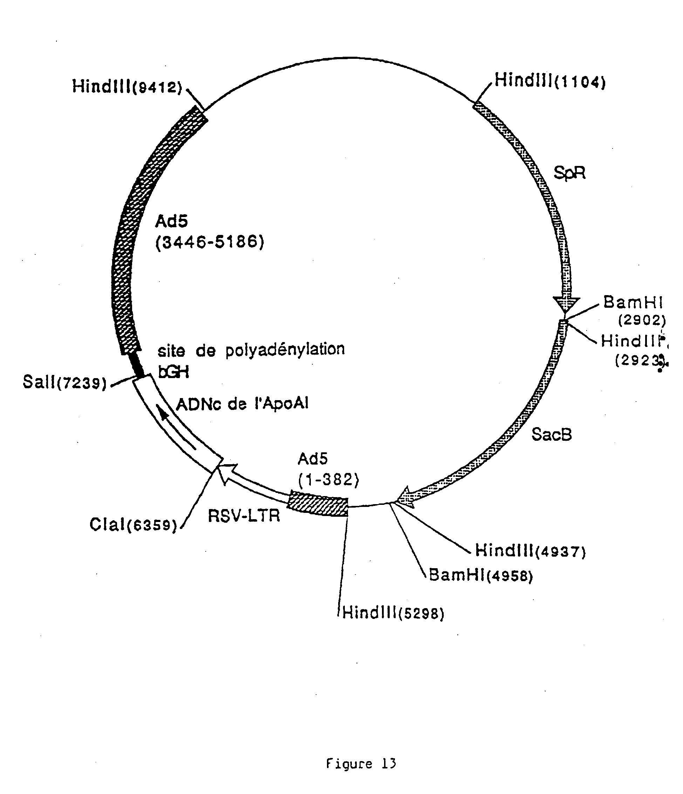

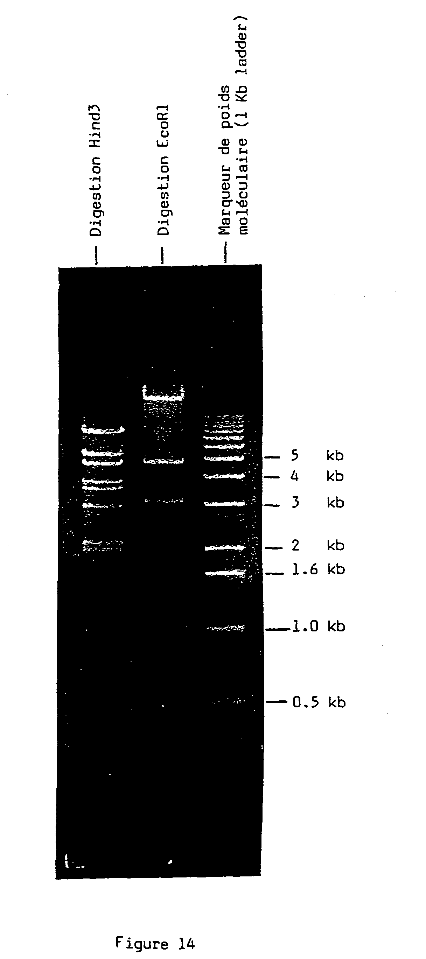

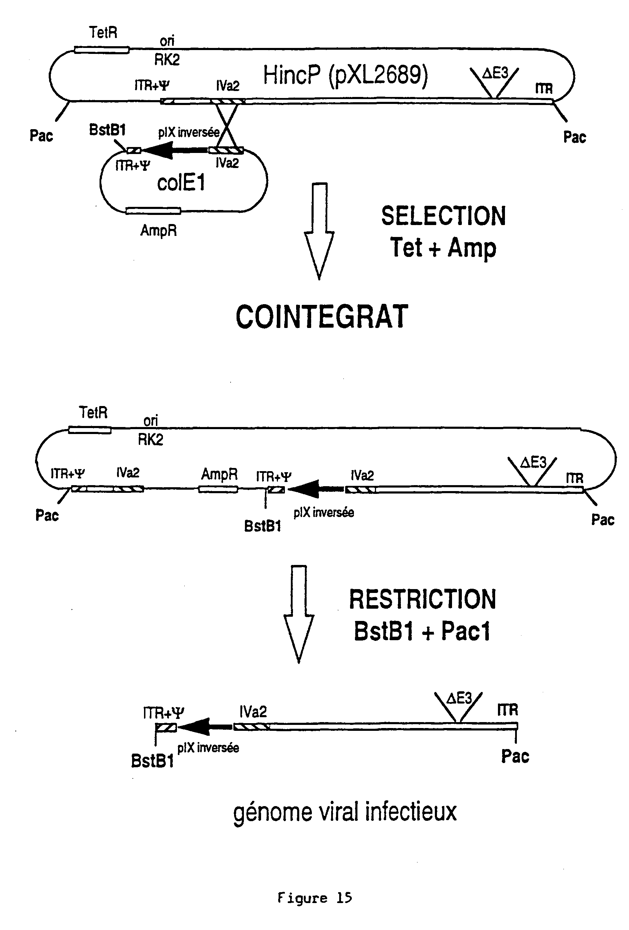

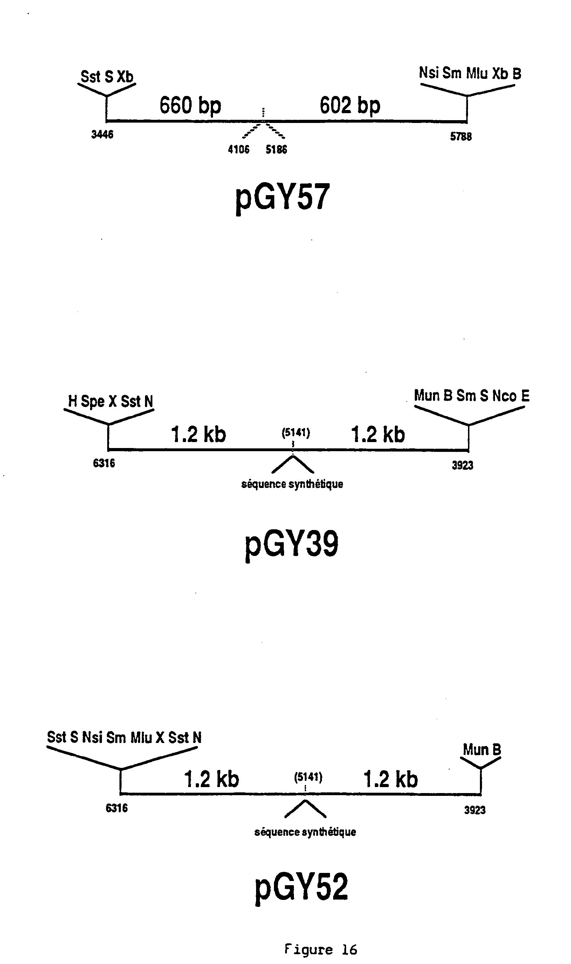

**Figure 3** : Construction du plasmide pXL2638.

Cettes construction est effectuée comme cela est décrit dans l'exemple 1-2, recombinaison homologue chez SF800 (E. coli polA). Amp^{r} : gène de résistance à l'ampicilline; pMB1 Ori, origine de réplication de pMB1; Tet^{r}, gène de résistance à la tétracycline: RK2 Ori, origine de réplication du plasmide RK2. Les parties circulaires des cartes des plasmides, les séquences adénovirales ne sont pas à la même échelle.

### Techniques générales de clonage, de biologie moléculaire.

Les méthodes classiques de biologie moléculaires telles que la centrifugation d'ADN plasmidique en gradient de chlorure de césium-bromure d'éthidium, les digestions par les enzymes de restriction, l'électrophorèse sur gel,la transformation dans E.coli, la précipitation des acides nucléiques etc, sont décrites dans la littérature (Maniatis et al.,1989).

Les enzymes ont été fournies par New-England Biolabs (Beverly, MA).
Pour les ligatures les fragments d'ADN sont séparés selon leur taille sur des gels d'agarose de 0.8 à 1,5 %, purifiés par GeneClean (BIO101, LaJolla CA) et incubés de nuit à 14°C dans un tampon Tris-HCl pH 7.4 50 mM, MgCl₂ 10 mM, DTT 10 mM, ATP 2 mM, en présence d'ADN ligase du phage T4.

Les ADN ligaturés sont utilisés pour transformer la souche rendue compétente E.coli TG1 [D(lac proA,B), supE, thi,hsdD5/ F traD36, proA⁺, B⁺, lacI^{q}, lacZDM15] (Maniatis et al.,1982) ou bien la souche E.coli polA SF800 (Heffron et al.,1977).
L'amplification par PCR, (Polymerase Chain Réaction), a également été réalisée selon Maniatis et al.,1989, avec les spécifications suivantes :
- Concentration en MgCl₂ portée à 8 mM.
- Température de dénaturation 95°C, température d'hybridation 55°C, température d'allongement 72°C. Ce cycle a été répété 25 fois dans un PE9600 Thermalcycler (Perkin Elmer, Norwalk CO).

Les oligonucléotides sont synthétisés en utilisant la chimie des phosphoramidites protégés en β par un groupement cyanoéthyl, (Sinha et al.,1984, Giles 1985), avec le synthétiseur automatique d'ADN Applied Biosystem modèle 394, (Applied Biosystem, Foster City CA), selon les recommandations du fabricant.
Le séquencage a été éffectué sur des matrices double-brin par la méthode de terminaison de chaînes en utilisant des amorces fluorescentes. Nous avons utilisé le kit se séquencage Taq Dye Primer Kit de chez Applied Biosystem (Applied Biosystem, Foster City CA) selon les spécifications du fabricant.

### Exemple 1 : Colonage d'un génome adénoviral, adénovirus humain du type 5, bordé de sites de restriction uniques sur un plasmide du groupe d'incompatibilité P se réplicant chez E.coli.

**Exemple 1-1** : Cet exemple illustre comment les extrémités du génome adénoviral peuvent être amplifiées par PCR, de manière à border les extrémités, (ITR), par un site PacI.

Afin d'obtenir des fréquences de recombinaison satisfaisantes par la suite, nous avons choisi des fragments d'environ 400 bp qui ont étés amplifiés.
Trois oligonucléotides ont été choisis :
- L'oligo 1 est commun aux deux amplifications puisqu'il correspond aux extrémités des ITR.

Les six premiers nucléotides constituent une queue qui évite la formation de structure secondaires. Les six nucléotides suivant constituent un site de restriction EcoRI qui est utilisé pour les étapes de clonage intermédiaires. Les huits nucléotides suivant créent un site PacI qui est absent du génome de l'Ad5 (la séquence de l'adénovirus Ad5 est accessible dans Genebank, mnémonique ADRCOMPGEN).
Les vingt-sept nucléotides suivant correspondent à l'extrémité gauche du génome de l'Ad5, position 1 à 27.

### Séquence de l'oligo 1 :

5'-CGGCGGGAATTCTTAATTAACATCATCAATAATATACCTTATTTTGG-3'SEQ ID N°1
------________
EcoRI  PacI

- L'oligo 3 utilisé avec l'oligo 1 permet d'amplifier un fragment de 384 bp à l'extrémité gauche. Il comprend une queue de six nucléotides. Les dix-huits nucléotides suivant créent les sites PstI, KpnI et SpeI. Les vingt-quatre nucléotides suivants correspondent à l'inverse-complémentaire de la séquence choisie sur l'Ad5 entre les positions 361 et 385.

### Séquence de l'oligo 3 :

5'-CACCACCTGCAGGGTACCACTAGTGTCTCCACGTAAACGGTCAAAGTC-3'SEQ ID N°3
PstI KpnI SpeI

La matrice choisie pour l'amplification de l'extrémité gauche est le plasmide pCLAI. Le plasmide pCLAI contient l'extrémité gauche sous forme d'un fragment EcoRI-XbaI de 454 bp cloné dans pIC19H.
- L'oligo 2 utilisé avec l'oligo 1 permet d'amplifier un fragment due 418 bp à l'extremité droite. Il comprend une queue de six nucléotides. Les six nucléotides suivant créent un site PstI. Les vingt-quatre nucléotides suivants correspondent à la séquence choisie sur l'Ad5 entre les positions 35517 et 35540.

### Séquence de l'oligo 2 :

5'-CACCACCTGCAGGGCAGCCATAACAGTCAGCCTTACC-3'SEQ ID N°2 PstI

La matrice choisie pour l'amplification de l'extrémité droite est le plasmide pY23. Le plasmide pY23 contient l'extrémité droite sous forme d'un Fragment AvrII-BclI de 470 bp cloné aux sites compatibles XbaI-BamHI de pIC19H.

Les produits de PCR ont étés déposés sur gel d'agarose. Les fragments de taille attendue ont été digérés par EcoRI et PstI, purifiés et ligaturés en présence de pUC19 digéré par EcoRI et PstI. Les deux plasmides recombinants obtenus ont été nommés pXL2623 pour l'extrémité gauche et pXL2624 pour l'extrémité droite. L'absence de mutation dans l'insert a été vérifiée par séquencage. Le fragment EcoRI-PstI de ces plasmides a été purifié comme précédemment décrit. Les deux fragments ont été mis ensemble à ligaturer dans pUC19 linéarisé par EcoRI. Le plasmide recombinant obtenu est nommé pXL2626, il contient les extrémités de l'Ad5 en tête-bêche. (Fig 2).

### Exemple 1-2 : Construction chez E.coli d'un plasmide contenant le génome adénoviral ΔE1,ΔE3, bordé par des sites PacI.

Le vecteur retenu pour recevoir le génome adenoviral est pRK290, (Ditta et al.,1980), qui appartient au groupe d'incompatibilité P. Ce vecteur se réplique chez les souches d'E.coli polA.

La stratégie d'intégration retenue est la recombinaison homologue dans la souche E.coli SF800 entre pXL2627 et le plasmide pFG144, (Graham et al., 1986).

Le plasmide pFG144 contient la totalité du génome de l'Ad5 moins deux délétions dans les régions E1 et E3. Il dérive de pMX2 et possède le gène de résistance à l'Ampicilline ainsi q'une origine de réplication pMB1 qui ne lui permet pas de se répliquer dans la souche E.coli SF800.

Dans un premier temps les extrémités tête-bêche du génome de l'Ad5 ont été clonées au site EcoRI unique de pRK290 selon le protocole déjà décrit pour la construction de pXL2626. Des cellules de la souche E.coli SF800 ont été rendues compétentes, transformées par pXL2627. Les cultures ont été étalées sur milieu LB en présence de tétracycline. A leur tour, les cellules issues d'un clone tétracycline résistant ont été rendues compétentes, transformées par le plasmide pFG144, puis étalées sur milieu LB en présence de tétracycline et d'ampicilline. Etant donné que ce plasmide ne se réplique pas dans la souche E.coli SF800, l'acquisition des résistances à la tétracycline et ampicilline ne peut se produire que par un évènement de recombinaison homologue entre les deux plasmides. En effet, ceux-ci ont en commun les extrémités droites, 418 bp, et gauches, 384 bp, du génome de l'Ad5. Les plasmides issus de ce premier évènement de recombinaison possèdent deux jeux d'extrémités. (Fig 2). Un second crossing-over, interne au plasmide peut donc avoir lieu. Dans ce cas, parmi les deux événements possibles, un seul conduit à la construction désirée, l'autre entraine la délétion complète du génome de l'Ad5 et la perte du gène de résistance à l'ampicilline Cette seconde recombinaison a été favorisée par une série de cultures dilution représentant 60 générations, en milieu LB supplementé par de la tétracycline et de l'ampicilline. L'ADN de clones isolés a été soumis à une analyse par Southern Blot. La sonde oligonucléotidique suivante a été synthétisée :
5'-CGTGGAGACACTAGTGGTACCCTGCAGGGCAGCCATA-3' SEQ ID N°4

Les bases 1 à 9 correspondent à la région gauche de la position 377 à 385 sur la séquence de l'Ad5. Les bases 28 à 36 correspondent à la séquence de la région droite entre les positions 35517 à 35525. Des digestions par les endonucléases PacI et ClaI, ou encore PacI et NdeI. ont montré la structure plasmidique attendue. Un clone correspondant à un tel évènement et ayant la structure plasmidique décrite Figure 3 à été retenu. Celui-ci a été nommé pXL2628.

pXL2628 a été digéré par XbaI, religaturé, puis transformé dans des cellules E.coli TG1 compétentes. Un clone Tet^{R} et Amp^{S} a été retenu. Son profil de restriction montre que la région correspondant au pMX2 a été délétée. Ce clone a été nommé pXL2638.
La digestion par PacI de pXL2638 libère le génome de l'Ad5 sous forme d'un fragment de 34 kb. Ce fragment peut être utilisé tel quel pour des expériences de transfection de cellules de mammifères transcomplémentantes pour les fonctions E1 de l'adénovirus.

### Exemple 2 : Production d'adénovirus recombinants

Des clones d'adénovirus recombinants peuvent être construits dans une première étape chez Escherichia coli, par inspections de fragments contenant un plusieurs gènes avec : des signaux de régulation appropriés pour exprimer ces gènes dans les cellules de mammifères étudiées, ou délétion de certains fragments du génome de adénovirus, ou encore la combinaison de ces deux événements, puis ensuite, après transfection de cellules productrices, un stock d'un tel virus recombinant peut être obtenu. Le plasmides pXL 2638 est purifié à partir d'une culture de cellules E. Coli TG1 compétentes transformées. Le génome adénoviral est libéré par digestion en présence de l'enzyme PacI. Le produit de digestion est utilisé directement pour produire les adénovirus recombinants. Pour cela, les cellules de la lignée 293 sont transfectées par le produit de digestion de pXL2638 en présence de phosphate de calcium. Les adénovirus recombinants produits sont ensuite sélectionnés par purification sur plaque Après isolement l'adénovirus recombinant est amplifié dans la lignée cellulaire 293, ce qui conduit à un surnageant de culture contenant l'adénovirus recombinant non purifié avant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'adénovirus peut être conservé à - 80°C dans 20 % de glycérol.

### Références bibliographiques :

Ausubel et al., 1987. Current protocols in molecular biology 1987-1988. John Willey and Sons, New York.
Bolivar et al., 1977. Gene 2:95.
Dagert et al., 1979. Gène, 6, 23-28.
Ditta et al., 1980. Plasmid,13, 149-154.
Ghosh-Choudhurry et al. 1986. Gene,50 ,161-171.
Hamilton et al., 1989. J. Bacteriol. 171:4617-4622.
Hanahan, D. 1983. J. Mol. Biol. 166:557.
Heffron et al., 1977. Proc.Natl.Acad.Sci. USA, 74, 702-706.
Maniatis T., et al. 1982. Molecular Cloning: A Laboratory Manual. Cold Spring Harbor laboratory, New York. Miller, et al., 1988. J. Bacteriol. 170:2575-2583.
Simoes, et al. 1991. New York Acad. Sci. 646:254-258.
Sinha N.D, et al. 1984. Nucl.Acids Res., 12, 4539-4557.
Slater, et al. 1993.. J. Bacteriol. 175:4260-4262.
Viera, et al.,1982. Gene, 19. 259-268.
Wirth, et al. 1989. Mol. Gen. Genet.. 216.175-177.
Zeef, et al. 1994. EMBO J. 13:5113-5120.

## Revendications

1. Plasmide procaryote comprenant un génome adénoviral complet recombinant, pouvant optionellement être dépourvu de tout ou partie de la région E1 et/ou de tout ou partie de la région E4 et/ou de tout ou partie de la région E3 et/ou de tout ou partie de la région IVa2, non interrompu par des régions du plasmide procaryote, bordé par un ou plusieurs sites de restriction non présent dans ledit génome, et bordé à droite et à gauche par des séquences ITR, les ITRs du génome n'étant pas jointes, et **caractérisé en ce qu'**il comprend une région permettant la réplication dans les cellules procaryotes, et une région permettant la sélection des cellules procaryotes contenant ledit plasmide.

2. Plasmide selon la revendication 1 **caractérisé en ce que** la région permettant la réplication comprend une origine de réplication fonctionnelle dans les cellules procaryotes.

3. Plasmide selon la revendication 2 **caractérisé en ce que** l'origine de réplication est issue d'un plasmide bactérien choisi parmi RK2, pBR322 et pUC.

4. Plasmide selon l'une des revendications précédentes **caractérisé en ce que** le génome adénoviral est dépourvu d'une partie de la région E1 allant des résidus 454 à 3328 ou 382 à 3446.

5. Plasmide selon l'une des revendications précédentes **caractérisé en ce que** le génome adénoviral est dépourvu d'une partie de la région E4 comprenant au moins les phases ORF3 et/ou ORF6.

6. Plasmide selon l'une des revendications précédentes **caractérisé en ce que** le génome adénoviral est d'origine humaine ou animale.

7. Plasmide selon la revendication 6 **caractérisé en ce que** le génome adénoviral est un génome adénoviral humain de type 2 ou 5.

8. Plasmide selon la revendication 7 **caractérisé en ce que** le génome adénoviral est un génome adénoviral canin choisi parmi le sérotype CAV2.

9. Plasmide selon l'une des revendications précédentes **caractérisé en ce que** le génome adénoviral recombinant dérive d'un adénovirus Ad5 et est bordé de sites Pacl, NspV ou Swal.

10. Plasmide selon l'une des revendications précédentes **caractérisé en ce que** le génome adénoviral recombinant comprend un acide nucléique d'intérêt.

11. Cellule procaryote contenant un plasmide selon l'une des revendications 1 à 10.

12. Procédé de production de génomes adénoviraux recombinants comprenant la culture de cellules procaryotes selon la revendication 11, et la récupération des plasmides.

13. Procédé selon la revendication 12 **caractérisé en ce que**, dans une étape supplémentaire, les plasmides sont traités de manière à exciser le génome adénoviral.

14. Procédé selon la revendication 13 **caractérisé en ce que** les plasmides sont traités en présence de l'enzyme de restriction correspondant aux sites bordant le génome adénoviral.

## Claims

1. Prokaryotic plasmid encompassing a complete recombinant adenoviral genome, which may optionally lack all or part of the E1 region and/or all or part of the E4 region and/or all or part of the E3 region and/or all or part of the IVa2 region, which is not interrupted by regions of the prokaryotic plasmid, which is flanked by one or more restriction sites which are not present in the said genome, and which is flanked on the right and on the left by ITR sequences, the ITRs of the genome not being joined, and **characterized in that** it encompasses a region which enables it to replicate in prokaryotic cells, and a region which enables the prokaryotic cells harbouring the said plasmid to be selected.

2. Plasmid according to Claim 1, **characterized in that** the region permitting replication encompasses an origin of replication which is functional in prokaryotic cells.

3. Plasmid according to Claim 2, **characterized in that** the origin of replication is derived from a bacterial plasmid selected from among RK2, pBR322 and pUC.

4. Plasmid according to one of the preceding claims, **characterized in that** the adenoviral genome lacks a part of the E1 region corresponding to residues 454 to 3328 or 382 to 3446.

5. Plasmid according to one of the preceding claims, **characterized in that** the adenoviral genome lacks a part of the E4 region which at least encompasses the ORF3 and/or ORF6 open reading frames.

6. Plasmid according to one of the preceding claims, **characterized in that** the adenoviral genome is of human or animal origin.

7. Plasmid according to Claim 6, **characterized in that** the adenoviral genome is a human type 2 or type 5 adenoviral genome.

8. Plasmid according to Claim 7, **characterized in that** the adenoviral genome is a canine adenoviral genome which is selected from the CAV2 serotype.

9. Plasmid according to one of the preceding claims, **characterized in that** the recombinant adenoviral genome is derived from an Ad5 adenovirus and is flanked by PacI, NspV or SwaI sites.

10. Plasmid according to one of the preceding claims, **characterized in that** the recombinant adenoviral genome encompasses a nucleic acid of interest.

11. Prokaryotic cell which contains a plasmid according to one of Claims 1 to 10.

12. Process for producing recombinant adenoviral genomes, comprising culturing prokaryotic cells according to Claim 11 and recovering the plasmids.

13. Process according to Claim 12, **characterized in that**, in a supplementary step, the plasmids are treated in such a way as to excise the adenoviral genome.

14. Process according to Claim 13, **characterized in that** the plasmids are treated in the presence of the restriction enzyme which corresponds to the sites flanking the adenoviral genome.

## Patentansprüche

1. Prokaryotisches Plasmid, das ein vollständiges rekombinantes Adenovirus-Genom enthält, in dem gegebenenfalls die gesamte oder ein Teil der E1-Region und/oder die gesamte oder ein Teil der E4-Region und/oder die gesamte oder ein Teil der E3-Region und/oder die gesamte oder ein Teil der IVa2-Region fehlen kann, das nicht durch Regionen des prokaryotischen Plasmids unterbrochen ist, durch eine oder mehrere, nicht in dem Genom vorhandene Restriktionsstellen flankiert wird und rechts und links von ITR-Sequenzen flankiert wird, wobei die ITRs des Genoms nicht verbunden sind, und **dadurch gekennzeichnet, dass** es eine Region enthält, welche die Replikation in prokaryotischen Zellen ermöglicht, und eine Region, welche die Selektion prokaryotischer Zellen, die das Plasmid enthalten, ermöglicht.

2. Plasmid nach Anspruch 1, **dadurch gekennzeichnet, dass** die Region, welche die Replikation ermöglicht, einen in prokaryotischen Zellen funktionsfähigen Replikationsursprung umfasst.

3. Plasmid nach Anspruch 2, **dadurch gekennzeichnet, dass** der Replikationsursprung aus einem bakteriellen Plasmid stammt, ausgewählt aus RK2, pBR322 und pUC.

4. Plasmid nach einem der vorhergehenden Ansprüche, **dadurch gekennzeichnet, dass** in dem Adenovirus-Genom ein Teil der E1-Region fehlt, der von den Resten 454 bis 3328 oder 382 bis 3446 reicht.

5. Plasmid nach einem der vorhergehenden Ansprüche, **dadurch gekennzeichnet, dass** in dem Adenovirus-Genom ein Teil der E4-Region fehlt, der mindestens die Leserahmen ORF3 und/oder ORF6 umfasst.

6. Plasmid nach einem der vorhergehenden Ansprüche, **dadurch gekennzeichnet, dass** das Adenovirus-Genom menschlichen oder tierischen Ursprungs ist.

7. Plasmid nach Anspruch 6, **dadurch gekennzeichnet, dass** das Adenovirus-Genom ein humanes Adenovirus-Genom des Typs 2 oder 5 ist.

8. Plasmid nach Anspruch 7, **dadurch gekennzeichnet, dass** das Adenovirus-Genom ein Hunde-Adenovirus-Genom ist, ausgewählt aus dem Serotyp CAV2.

9. Plasmid nach einem der vorhergehenden Ansprüche, **dadurch gekennzeichnet, dass** das rekombinante Adenovirus-Genom von einem Ad5-Adenovirus stammt und von PacI-, NspV- oder SwaI-Stellen flankiert wird.

10. Plasmid nach einem der vorhergehenden Ansprüche, **dadurch gekennzeichnet, dass** das rekombinante Adenovirus-Genom eine interessierende Nukleinsäure umfasst.

11. Prokaryotische Zelle, die ein Plasmid nach einem der Ansprüche 1 bis 10 enthält.

12. Verfahren zur Herstellung rekombinanter Adenovirus-Genome, umfassend die Kultur prokaryotischer Zellen nach Anspruch 11 und die Gewinnung der Plasmide.

13. Verfahren nach Anspruch 12, **dadurch gekennzeichnet, dass** in einem zusätzlichen Schritt die Plasmide derart behandelt werden, dass das Adenovirus-Genom ausgeschnitten wird.

14. Verfahren nach Anspruch 13, **dadurch gekennzeichnet, dass** die Plasmide in Gegenwart des Restriktionsenzyms behandelt werden, das den Stellen entspricht, die das Adenovirus-Genom flankieren.


### LISTE DE SEQUENCES

(1) INFORMATIONS GENERALES:
   (i) DEPOSANT:
      (A) NOM: RHONE POULENC RORER
      (B) RUE: 20 AVENUE RAYMOND ARON
      (C) VILLE: ANTONY CEDEX
      (E) PAYS: FRANCE
      (F) CODE POSTAL: 92165
      (G) TELEPHONE: 40.91.69.22
      (H) TELECOPIE: 40.91.72.91
   (ii) TITRE DE L'INVENTION:_PROCEDE DE PREPARATION DE GENOME D'ADENOVIRUS RECOMBINANTS
   (iii) NOMBRE DE SEQUENCES:4
   (iv) FORME DECHIFFRABLE PAR ORDINATEUR:
      (A) TYPE DE SUPPORT: Tape
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 47 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
      CGGCGGGAAT TCTTAATTAA CATCATCAAT AATATACCTT ATTTTGG
(2) INFORMATIONS POUR LA SEQ ID NO: 2:
   (i) CARACTERISTIQUES DE LA SEQUENCE:
      (A) LONGUEUR: 37 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
      CACCACCTGC AGGGCAGCCA TAACAGTCAG CCTTACC
(2) INFORMATIONS POUR LA SEQ ID NO: 3:
   (i) CARACTERISTIQUES DE LA SEQUENCE:
      (A) LONGUEUR: 48 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 3:
      CACCACCTGC AGGGTACCAC TAGTGTCTCC ACGTAAACGG TCAAAGTC
(2) INFORMATIONS POUR LA SEQ ID NO: 4:
   (i) CARACTERISTIQUES DE LA SEQUENCE:
      (A) LONGUEUR: 37 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
      CGTGGAGACA CTAGTGGTAC CCTGCAGGGC AGCCATA